# EUROPEAN PATENT APPLICATION

(11) **EP 4 704 096 A1**
(43) Date of publication of application: **04.03.2026**
(21) Application number: 23935401.2
(22) Date of filing: 28.04.2023
(51) Int. Cl.: G16H 10/00

(54) **MANAGEMENT SERVER, POLICY MANAGEMENT METHOD, AND POLICY MANAGEMENT PROGRAM**

(71) Applicant: NEC Corporation, 108-8001 Tokyo (JP)
(72) Inventor: TANAKA, Yuki, Tokyo 108-8001 (JP); ONOUE, Kousuke, Tokyo 108-8001 (JP)
(74) Representative: Betten & Resch
(86) International application number: PCT/JP2023/016931
(87) International publication number: WO 2024/224625

(57) **Abstract**

The present invention contributes to application of appropriate management policy/policies by each of various participants involved in personalized medical process(es). A policy management server for managing rule(s) under which a plurality of participants jointly handle genomic data, includes a policy database that stores policies to be applied separately for each of the plurality of participants.

## Description

### TECHNICAL FIELD

The present invention relates to a management server, policy management method, and policy management program.

### BACKGROUND

Conventionally, medicine has been centered on disease, with its primary aim being to investigate the causes of illnesses and to develop treatments therefor. It has long been known that applying the same treatment to the same disease is not always appropriate; however, individual differences in treatment efficacy could only be understood by observing the treatment and its effects, making it difficult to devise an optimal treatment plan for each individual.

Meanwhile, advancements in technologies such as DNA sequencing and the identification of individual-specific single nucleotide polymorphisms (SNPs) have made it possible to observe how one person differs from another. As a result, it is becoming increasingly feasible to use such information to plan treatment methods optimized for individual patients. For instance, Patent Literature 1 describes a trusted privacy protection method for processing and handling tests related to human genome information.

### CITATION LIST

### PATENT LITERATURE

[Patent Literature 1]
Japanese Patent Kohyo Publication No. JP2015-502588A

### SUMMARY

### TECHNICAL PROBLEM

The disclosure of the literature cited above is incorporated herein in its entirety by reference thereto. The following analysis is provided by the inventors of the present invention.

Genomic data that represents DNA base sequences is generally regarded, in societal terms, as a "personally identifiable code" and therefore must be managed strictly as personal information. Further, medically significant "genomic information" obtained by adding to genomic data interpretations such as susceptibility to disease or selection of therapeutic drugs is considered sensitive personal information and requires particularly careful handling.

Meanwhile, in personalized medicine, it is difficult to complete all processes solely within a hospital or within a specific network. Obtaining genomic data or genomic information as digital data from patient samples requires a specialized apparatus such as a next-generation sequencer, and it has already become common practice for hospitals to outsource sequencing tasks to external companies equipped with such an apparatus. Consequently, it is challenging to carry out data exchanges with companies that perform sequencing entirely within a hospital's network. In addition to sequencers, personalized medicine requires building dedicated servers used to analyze genomic data and genomic information as well as servers for conducting patient-specific drug development, such as neoantigen prediction, using technologies like AI based on genomic data and genomic information. However, it is not cost-effective for each hospital to consistently maintain these facilities. Outsourcing the processes to external entities is more cost-efficient, and employing a multi-vendor approach to handle such tasks is a rational solution. Therefore, genomic data and genomic information not only require strict management as personal information, but also must be handled within a scope of their designated roles by each of various participating companies involved in personalized medical processes. In other words, it is not sufficient to treat genomic data and genomic information merely as critical information to be tightly controlled within a closed environment; rather, it is necessary for each of the various participating companies involved in the processes to apply management policy.

In view of the above problem, it is an object of the present invention to provide a management server, policy management method, and policy management program that contribute to application of appropriate management policy/policies by each of various participants involved in personalized medical process(es).

### SOLUTION TO PROBLEM

According to a first aspect of the present invention, there is provided a policy management server for managing rule(s) under which a plurality of participants jointly handle genomic data, the management server including a policy database that stores policies to be applied separately for each of the plurality of participants.

According to a second aspect of the present invention, there is provided a policy management method including: registering, in a policy database, policies to be applied separately for each of a plurality of participants; and inquiring of a management server whether handling of genomic data executed on an information processing apparatus used by the plurality of participants to handle the genomic data violates a policy/policies, wherein the management server determines whether or not handling of the genomic data in the information processing apparatus violates a policy/policies while referring to the policy database.

According to a third aspect of the present invention, there is provided a policy management program executed on a policy management server that manages rule(s) under which a plurality of participants jointly handle genomic data, the policy management program causing the management server to execute: a process of registering, in a policy database, policies to be applied separately for each of the plurality of participants; a process of receiving an inquiry/inquiries as to whether handling of the genomic data executed on an information processing apparatus used by the plurality of participants to handle the genomic data violates a policy/policies; and a process of determining whether or not handling of the genomic data in the information processing apparatus violates a policy/policies while referring to the policy database.
Further, the program(s) can be stored in a computer-readable storage medium. The storage medium may be a non-transitory one such as a semiconductor memory, a hard disk, a magnetic recording medium, an optical recording medium, and the like. The present invention can also be realized as a computer program product.

### ADVANTAGEOUS EFFECTS OF INVENTION

According to each aspect of the present invention, there can be provided a management server, policy management method, and policy management program that contribute to application of appropriate management policy/policies by each of various participants involved in personalized medical process(es).

### BRIEF DESCRIPTION OF DRAWINGS

[Fig. 1] Fig. 1 is a diagram illustrating a flow of genomic data in personalized medicine.
[Fig. 2] Fig. 2 is a schematic diagram illustrating application of management policy/policies using a management server relating to an example embodiment.
[Fig. 3] Fig. 3 is a diagram showing an example of policy information stored in a policy database.
[Fig. 4] Fig. 4 is a diagram illustrating a flow of a policy management method relating to the example embodiment.
[Fig. 5] Fig. 5 is a diagram illustrating a hardware configuration example of the management server used in the example embodiment.

### EXAMPLE EMBODIMENTS

The following describes an example embodiment of the present invention with reference to the drawings. The present invention, however, is not limited to the example embodiment described below. Further, in each drawing, the same or corresponding elements are appropriately designated by the same reference signs. It should be noted that the drawings are schematic, and the dimensional relationships and the ratios between the elements may differ from the actual ones. The dimensional relationships and the ratios between the drawings may also be different in some sections.

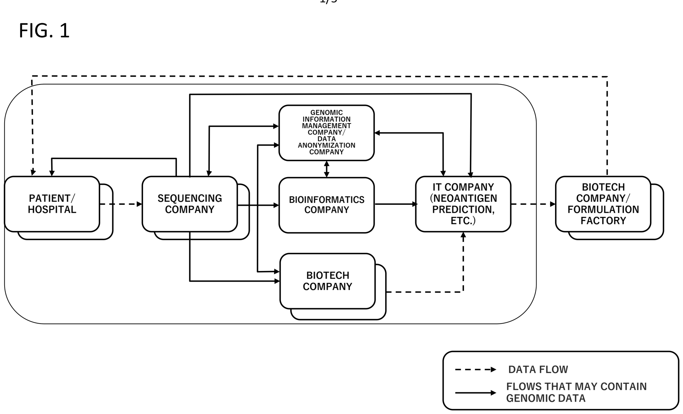

Fig. 1 is a diagram illustrating a flow of genomic data in personalized medicine. As shown in Fig. 1, genomic data provided by a patient is not confined within a hospital but is exchanged among various participating companies. As previously stated, all companies involved in processing of genomic data must apply management policy/policies with respect to personal information and sensitive personal information. Moreover, each company participating in the processing of genomic data must handle the data strictly within a scope of its designated role.

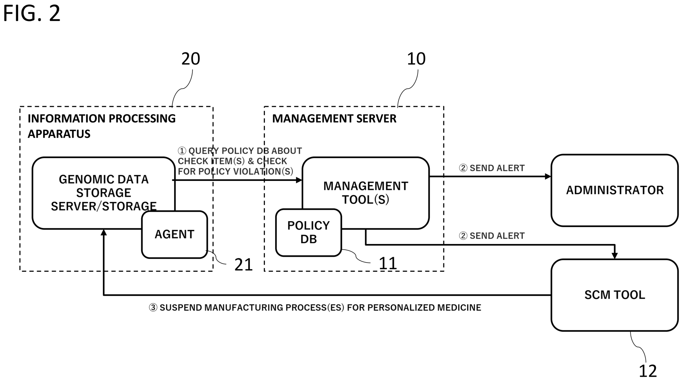

Fig. 2 is a schematic diagram illustrating application of management policy/policies using a management server relating to an example embodiment. As shown in Fig. 2, the application of management policy/policies is realized by the management server 10. The management server includes a policy database 11 that stores policies to be applied separately for each of a plurality of participants, and manages rule(s) under which the plurality of participants jointly handle genomic data. Meanwhile, an agent program 21, capable of communicating with a management tool(s) installed on the management server 10, is installed on an information processing apparatus 20, used by the plurality of participants to handle genomic data.

The agent program 21 transmits a handling state(s) of genomic data stored in the information processing apparatus 20 to the management server 10, and the management server 10 determines whether the handling of the genomic data on the information processing apparatus 20 complies with the policy/policies by referring to the policy database 11. The policy database 11 stores policies to be applied separately for each of the plurality of participants.

If it is determined that the handling of the genomic data on the information processing apparatus 20 violates the policy/policies, the management server 10 executes a process of suspending a manufacturing process(es) for personalized medicine. For instance, as shown in Fig. 2, the management server 10 may be configured to notify an alert to an SCM (supply chain management) tool 12 so that the SCM tool 12 executes the process of suspending the manufacturing process(es) for personalized medicine. Note that it is also possible to suspend not only the manufacturing process(es) for personalized medicine by the information processing device 20 that committed the policy violation, but also manufacturing process(es) of downstream participant(s) in the manufacturing process(es) for personalized medicine.

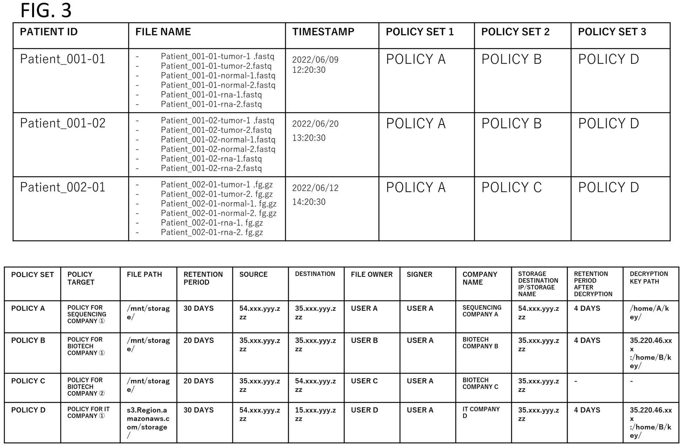

Fig. 3 is a diagram showing an example of the policy information stored in the policy database. As shown in Fig. 3, the policy database 11 stores policies to be applied separately for each of a plurality of participants. More specifically, the policy database 11 defines policy set(s) to be applied for each genomic dataset (for each Patient ID), and each policy set specifies applicable policy target(s) (participating company/companies). The management server 10 determines whether a handling state(s) of genomic data received from the agent program 21 violates any rule in policy set(s).

The agent program 21 determines whether or not data stored in the information processing apparatus 20 is genomic data by checking the data's file extension or by reading part of the file using commands such as "head" or "more" to verify whether it conforms to a genome-related file format.
1. Check whether the file is in a format indicating genomic data or genomic information, such as fasta, fastq, or vcf format.
2. Check whether the data is whole genome sequencing data, whole exome sequencing data, whole-genome single nucleotide polymorphism (SNP) data, sequence data constituted by 40 or more SNPs independent of each other, or a short tandem repeat (STR) of a four-base unit at nine or more loci. If the data meets any of the above conditions, classify it as a personal information identifier.
3. Check whether annotation information is appended. If so, classify the data as sensitive personal information. If not, classify it as a personal information identifier.

For instance, the agent program 21 transmits the following questions to the management server 10 to inquire about potential policy violation(s).
- Whether permissions are set appropriately (for instance, whether file(s) related to genomic data or genomic information are set to read-only).
- Whether a checksum value of file(s) related to genomic data or genomic information is obtained periodically, and whether there is any change in the checksum value.
- Analyze file access log(s) to determine whether any suspicious file access has occurred.
- Analyze server/storage access log(s) to determine whether any suspicious access has occurred.
- Whether file(s) related to genomic data or genomic information are signed.
- Whether file(s) related to genomic data or genomic information are encrypted.
- Whether there is any file that can be used to identify personal information by linking it to file(s) related to genomic data or genomic information (for instance, search for .csv, .tsv, or .xlsx files on a storage server and check whether they contain any ID that can be used to identify an individual by linking it to any genome-related file name or any genome-related file).
- [Policy DB verification] Whether genomic file name(s) are appropriate. Whether it has been altered on an agent server side.
- [Policy DB verification] Whether retention period(s) are appropriate. Whether file(s) still exist beyond the appropriate retention period(s).
- [Policy DB verification] Whether any file has been forwarded to/from any destination/source other than those permitted.
- [Policy DB verification] Whether any file timestamp has been altered.
- [Policy DB verification] Whether file owner(s) match registered owner(s).
- [Policy DB verification] Whether file signer(s) are appropriate.
- [Policy DB verification] In a case where a domain exists, whether it matches a storage destination owner.
- [Policy DB verification] Whether a storage destination IP matches.
- [Policy DB verification] Whether checksum value(s) match registered value(s).
- [Policy DB verification] Whether any file exists beyond a retention period after decryption.
- [Policy DB verification] Whether file path(s) after decryption are set to pre-specified path(s).

In the above description, the agent program 21 transmits a handling state(s) of genomic data stored in the information processing apparatus 20 to the management server 10, and the management server 10 determines whether the handling of the genomic data in the information processing apparatus 20 violates a policy/policies while referring to the policy database 11. However, the information processing apparatus 20 may distribute the management policy/policies stored in the policy database 11 to the information processing apparatus 20, and the agent program 21 may monitor for policy violation(s) by referring to the distributed management policy/policies.

If a policy violation(s) is found as a result of determining the compliance of the management policy/policies as described above, the management server 10 may notify an alert to an administrator. Further, the management server 10 may also notify the alert to an external apparatus, such as an SCM tool, which may stop a process(es) that violates the policy/policies.

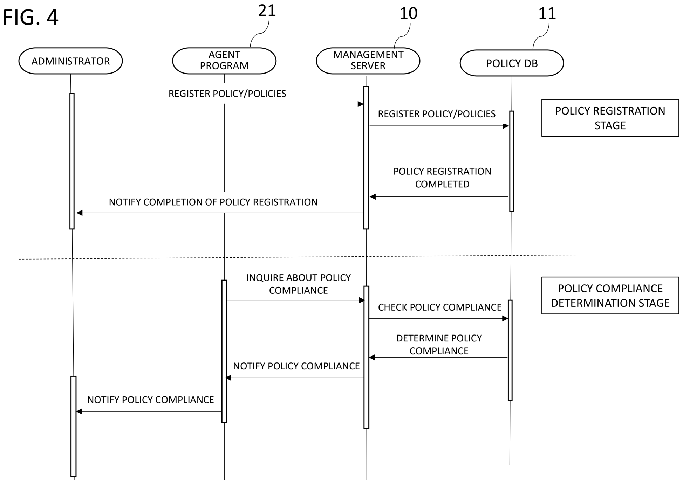

Fig. 4 is a diagram illustrating a flow of a policy management method relating to the example embodiment. As shown in Fig. 4, the policy management method is divided into a policy registration stage and a policy compliance determination stage. In the policy registration stage, the administrator registers policy/policies in the policy database via the management server. When the policy registration in the policy database is completed, the management server notifies the administrator of the completion of the policy registration.

In the policy compliance determination stage, the agent program 21 transmits a handling state(s) of genomic data stored in the information processing apparatus 20 to the management server 10 and inquires whether the handling of the genomic data in the information processing apparatus 20 violates any policy. The management server 10 determines whether or not the handling of the genomic data in the information processing apparatus 20 violates any policy while referring to the policy database 11. Upon obtaining a result(s) of the policy compliance determination, the management server 10 notifies the agent program 21 of the policy compliance determination result(s). The agent program 21, having received the policy compliance determination result(s), notifies the administrator of the result(s). If the policy compliance determination result(s) indicates a policy violation(s), the administrator executes corrective measure(s) to remedy the policy violation(s).

### (Hardware Configuration)

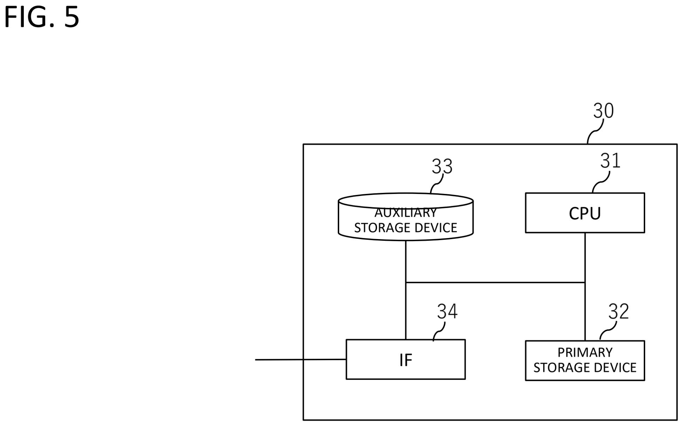

Fig. 5 is a diagram illustrating a hardware configuration example of the management server used in the example embodiment. In other words, the management server 10 is able to achieve each function thereof by causing an information processing apparatus (computer) 30 employing the hardware configuration shown in Fig. 5 to execute the policy management method described above as a program(s). It should be noted that the hardware configuration example shown in Fig. 5 is merely an example of the hardware configuration that achieves each function of the management server 10 and is not intended to limit the hardware configuration of the management server 10. The management server 10 may include hardware not shown in Fig. 5.

As shown in Fig. 5, the hardware configuration that may be employed by the management server 10 includes a CPU (Central Processing Unit) 31, a primary storage device 32, an auxiliary storage device 33, and an IF (interface) part 34, which are connected to each other by, for instance, an internal bus.

The CPU 31 executes each instruction included in the policy management program executed by the information processing apparatus (computer) 30. The primary storage device 32 is, for instance, a RAM (Random Access Memory) and temporarily stores various programs such as the policy management program executed by the information processing apparatus (computer) 30 so that the CPU 31 can process the program(s).

The auxiliary storage device 33 is, for instance, an HDD (Hard Disk Drive) and is capable of storing various programs, such as the policy management program executed by the information processing apparatus (computer) 30, in the medium to long term. Various programs such as the policy management program may be provided as a program product(s) stored in a non-transitory computer-readable storage medium.

The IF part 34 provides an interface related to, for instance, an input and output of the management server 10.

The information processing apparatus (computer) 30 employing the hardware configuration described above achieves each function of the management server 10 by executing the flow visualization method described above as a program(s).

Part or entirety of the example embodiment above can be described as (but not limited to) the following Supplementary Notes.

### [Supplementary Note 1]

A policy management server for managing rule(s) under which a plurality of participants jointly handle genomic data, the management server comprising:
a policy database that stores policies to be applied separately for each of the plurality of participants.

### [Supplementary Note 2]

The management server according to Supplementary Note 1, determining whether or not handling of the genomic data in the information processing apparatus violates a policy/policies while referring to the policy database, wherein the handling is inquired by an agent program executed on an information processing apparatus used by the plurality of participants to handle genomic data.

### [Supplementary Note 3]

The management server according to Supplementary Note 2, performing a process of suspending a manufacturing process(es) for personalized medicine when determining that the handling of the genomic data violates a policy/policies.

### [Supplementary Note 4]

The management server according to Supplementary Note 1, distributing policies to be applied for each of the plurality of participants to an agent program executed on an information processing apparatus used by the plurality of participants to handle genomic data, wherein
the agent program monitors handling of the genomic data in accordance with the distributed policy/policies.

### [Supplementary Note 5]

The management server according to any one of Supplementary Notes 2 to 4, wherein the agent program determines whether or not data held by the information processing apparatus is genomic data based on the data's file extension or whether file content of the data conforms to a genome-related file format.

### [Supplementary Note 6]

A policy management method, comprising:
registering, in a policy database, policies to be applied separately for each of a plurality of participants; and
inquiring of a management server whether handling of genomic data executed on an information processing apparatus used by the plurality of participants to handle the genomic data violates a policy/policies,
wherein
the management server determines whether or not handling of the genomic data in the information processing apparatus violates a policy/policies while referring to the policy database.

### [Supplementary Note 7]

The policy management method according to Supplementary Note 6, determining whether or not data held by the information processing apparatus is genomic data based on the data's file extension or whether file content of the data conforms to a genome-related file format.

### [Supplementary Note 8]

A policy management program, executed on a policy management server that manages rule(s) under which a plurality of participants jointly handle genomic data, the policy management program causing the management server to execute:
a process of registering, in a policy database, policies to be applied separately for each of the plurality of participants;
a process of receiving an inquiry/inquiries as to whether handling of the genomic data executed on an information processing apparatus used by the plurality of participants to handle the genomic data violates a policy/policies; and
a process of determining whether or not handling of the genomic data in the information processing apparatus violates a policy/policies while referring to the policy database.

Further, the disclosure of Patent Literature cited above is incorporated herein in its entirety by reference thereto. It is to be noted that it is possible to modify or adjust the example embodiments or examples within the scope of the whole disclosure of the present invention (including Claims) and based on the basic technical concept thereof. Further, it is possible to variously combine or select (or partially omit) a wide variety of the disclosed elements (including the individual elements of the individual claims, the individual elements of the individual example embodiments or examples, and the individual elements of the individual figures) within the scope of the whole disclosure of the present invention. That is, it is self-explanatory that the present invention includes any types of variations and modifications to be done by a skilled person according to the whole disclosure including Claims, and the technical concept of the present invention. Particularly, any numerical values or ranges disclosed herein should be interpreted that any intermediate values or subranges falling within the disclosed ranges are also disclosed even without explicit recital thereof. In addition, using some or all of the disclosed elements in each literature cited above as necessary in combination with the elements described herein as part of the disclosure of the present invention in accordance with the object of the present invention shall be considered to be included in the disclosed elements of the present application.

### REFERENCE SIGNS LIST

10: management server
11: policy database
12: SCM tool
20: information processing apparatus
21: agent program
30: information processing apparatus
31: CPU
32: primary storage device
33: auxiliary storage device
34: IF part

## Claims

1. A policy management server for managing rule(s) under which a plurality of participants jointly handle genomic data, the management server comprising:
a policy database that stores policies to be applied separately for each of the plurality of participants.

2. The management server according to Claim 1, determining whether or not handling of the genomic data in the information processing apparatus violates a policy/policies while referring to the policy database, wherein the handling is inquired by an agent program executed on an information processing apparatus used by the plurality of participants to handle genomic data.

3. The management server according to Claim 2, performing a process of suspending a manufacturing process(es) for personalized medicine when determining that the handling of the genomic data violates a policy/policies.

4. The management server according to Claim 1, distributing policies to be applied for each of the plurality of participants to an agent program executed on an information processing apparatus used by the plurality of participants to handle genomic data, wherein
the agent program monitors handling of the genomic data in accordance with the distributed policy/policies.

5. The management server according to any one of Claims 2 to 4, wherein the agent program determines whether or not data held by the information processing apparatus is genomic data based on the data's file extension or whether file content of the data conforms to a genome-related file format.

6. A policy management method, comprising:
registering, in a policy database, policies to be applied separately for each of a plurality of participants; and
inquiring of a management server whether handling of genomic data executed on an information processing apparatus used by the plurality of participants to handle the genomic data violates a policy/policies, wherein
the management server determines whether or not handling of the genomic data in the information processing apparatus violates a policy/policies while referring to the policy database.

7. The policy management method according to Claim 6, determining whether or not data held by the information processing apparatus is genomic data based on the data's file extension or whether file content of the data conforms to a genome-related file format.

8. A policy management program, executed on a policy management server that manages rule(s) under which a plurality of participants jointly handle genomic data, the policy management program causing the management server to execute:
a process of registering, in a policy database, policies to be applied separately for each of the plurality of participants;
a process of receiving an inquiry/inquiries as to whether handling of the genomic data executed on an information processing apparatus used by the plurality of participants to handle the genomic data violates a policy/policies; and
a process of determining whether or not handling of the genomic data in the information processing apparatus violates a policy/policies while referring to the policy database.
